(19) Europäisches Patentamt
European Patent Office
Office européen des brevets

(11) Publication number: **0 155 244**
**A2**

(12) **EUROPEAN PATENT APPLICATION**

(21) Application number: **85830060.1**

(22) Date of filing: **08.03.85**

(51) Int. Cl.⁴: **C 07 D 401/04**, A 61 K 31/47
// C07D207/32

(30) Priority: **13.03.84 IT 4784884**

(43) Date of publication of application: **18.09.85**
**Bulletin 85/38**

(84) Designated Contracting States: **AT BE CH DE FR GB LI LU NL SE**

(71) Applicant: **BIOCHEM DESIGN S.r.l., Via Portuense 216, I-00149 Roma (IT)**

(72) Inventor: **Sibilia, Luigi, Via Monti Parioli 49A, I-00197 Roma (IT)**

(74) Representative: **Sarpi, Maurizio, Studio FERRARIO Via Collina, 36, I-00187 Roma (IT)**

(54) **1-Ethyl-6-fluoro-7-(1H-pirrol-1-yl)-1,4-dihydro-4-oxoquinoline-3-carboxylic acid: preparation and antimicrobial activities.**

(57) The title compound 1-ethyl-6-fluoro-7-(1-pyrrol-1-yl)-1,4-dihydro-4-oxoquinoline-3-carboxylic acid is a quinolone carboxylic acid derivative of "oxacin" family bearing a fluorine atom and a pyrrol-1-yl moiety at 6 and 7 position respectively. It is represented by the following formula:

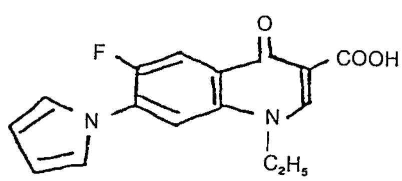

and has been prepared by alkaline hydrolysis of the corresponding ethyl ester. The latter compound has been synthesized starting from 2-fluoro-5-nitroaniline. Treatment of this amine with diethoxytetrahydrofuran in glacial acetic acid afforded 4-fluoro-3-(1-H-pyrrol-1-yl)-nitrobenzene, which was then reduced by catalytic hydrogenation to 4-fluoro-3-(1H-pyrrol-1-yl)aniline.

Reaction of this compound with diethyl ethoxymethylene malonate and cyclization of the malonate obtained by heating in diphenyl ether at 120° C resulted in formation of ethyl ester of 6-fluoro-7-(1H-pyrrol-1-yl)quinoline-3-carboxylic acid. Ethylation of this ester with ethyl iodide in the presence of sodium carbonate gave the required 1-ethyl-6-fluoro-7-(1H-pyrrol-1-yl)-1,4-dihydro-4-oxoquinoline-3-carboxylic acid ethyl ester.

The title acid exhibits high activity against Gram-positive bacteria, Enterobacteriaceae, Proteus and Pseudomonas, superior to those of nalidixic acid, piromidic acid and pipemidic acid; again, its antimicrobial spectrum is somewhat superior also to that of enoxacin.

1-Ethyl-6-fluoro-7-(1H-pyrrol-1-yl)-1,4-dihydro-4-oxoquinoline-3-carboxylic acid is a new potent fluorinated quinolone antibacterial agent useful in the management of urinary tract and systemic infections.

The present invention also relates to a process for the preparation of such compound.

EP 0 155 244 A2

1-ETHYL-6-FLUORO-7-(1-H-PYRROL-1-YL)-1,4-DIHYDRO-4-OXOQUINOLINE -3-CARBOXYLIC ACID : PREPARATION AND ANTIMICROBIAL ACTIVITIES.

This invention relates to 1-ethyl-6-fluoro-7--(1H-pyrrol-1-yl)-1,4-dihydro-4-oxoquinoline-3-carboxylic acid, a new potent antibacterial agent represented by the following formula (1).

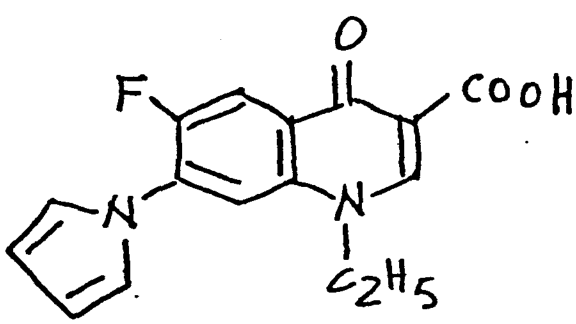

(1)

A large number of publications on the antimicrobial activities of quinolone and other "nalidixic acid analogues " are now available.The special interest in the activity of gyrase inhibitors started with the discovery of nalidixic acid (2) in 1964 (U.S.Pat.3.149.104,Sept.16,1964-;G.Y.Lesher and M.D.Gruett,Sterling Drug,Inc.).

Nalidixic acid has a minimal activity against many clinically important pathogens and has to be given in

high dose,resulting in untoward effects.

Agents developed subsequent to nalidixic acid were oxolinic acid (3) (U.S.Pat.3.287.458,Nov.22,1966;D.Kaminsky and R.I.Meltzer,Warner-Lambart Co.),piromidic acid (4) (U.S.Pat.3.673.184,June 27,1972;S.Minami,T.Shono, M.Shimizu and Y.Takase,Dainippon Pharmaceutical Co.Ltd.) and pipemidic acid (5) (U.S.Pat.3.887.557,June 3,1975;S. Minami,J.Matamoto,K.Kawaguchi,S.Michio,M.Shimizu,Y.Takase and S.Nakamuro,Dainippon Pharmaceutical Co.Ltd.).

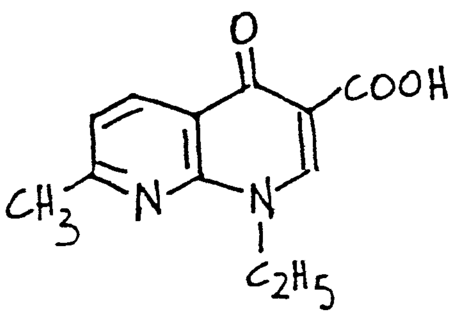

(2)

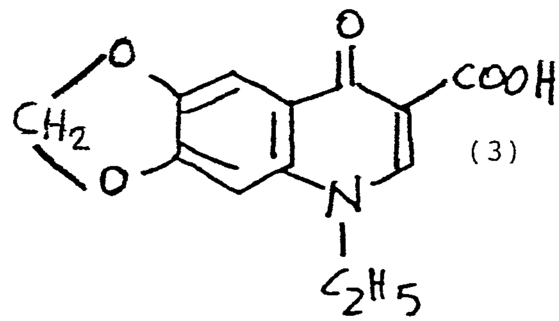

(3)

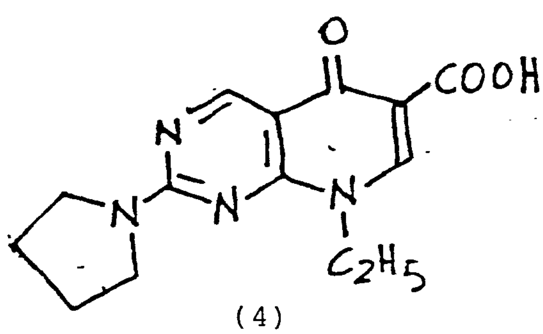

(4)

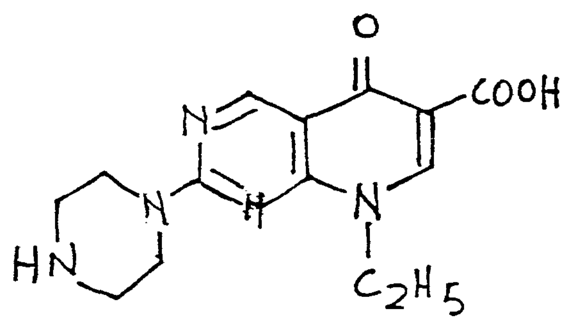

(5)

The more recent third generation of "oxacin" family includes many new gyrase inhibitors with 4-quinolone moiety and other structurally related compounds.The most important members are ciprofloxacin,pefloxacin and norfloxacin among 4-quinolones,flumequine and ofloxacin in the tricyclic quinolone class and enoxacin among naphthyridine derivates related to nalidixic acid.They can be represented by the general formulas 6,7,and 8.

These compounds possess high in vitro antibacterial activities and are characterized by sufficiently high concentrations in serum and in tissues.Therefore a potential use for these new drug's could be the treatment of systemic infections.

Compound (1),with structural features resemb - ling those of piromidic acid (4) and norfloxacin (6; R=H , R'=$C_2H_5$),

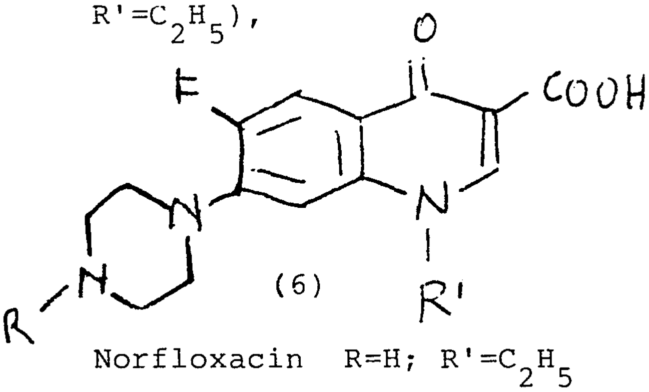

(6)

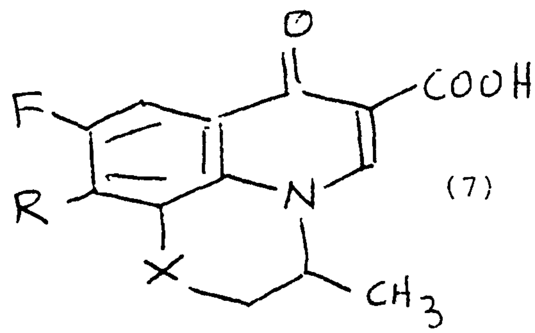

(7)

Norfloxacin R=H; R'=$C_2H_5$

Pefloxacin R=$CH_3$ R'=$C_2H_5$

Ciprofloxacin R=H;R'= 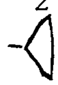

Flumequine R=H; X=$CH_2$

Ofloxacin R=-N 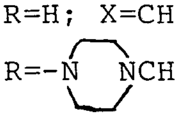 N$CH_3$;X=O

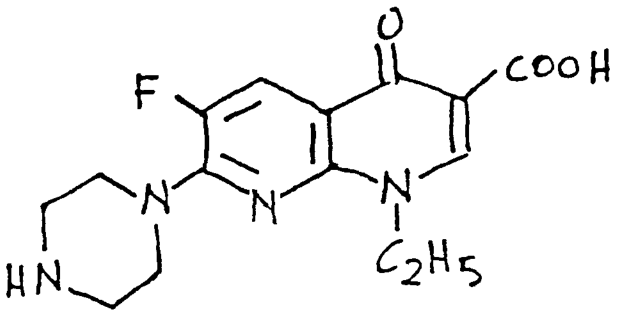

(8) Enoxacin

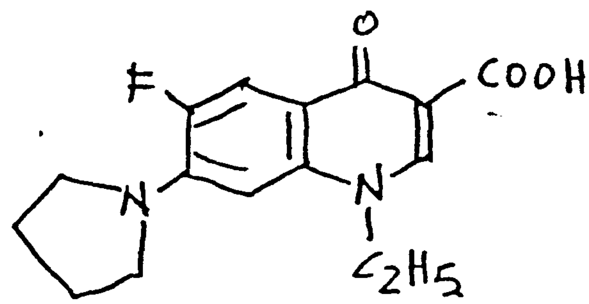

(9)

was expected to show high broad-spectrum antibacterial activities.These expectations were confirmed by data of

screening against microorganisms,which showed that deriva tive (1) is greatly superior in antimicrobial potency to nalidixic acid,piromidic acid,pipemidic acid and sometimes superior also to norfloxacin.Catalytic reduction of (1) led to the know pyrrolidinyl derivative (9),which showed a lower antimicrobial activity.

The process for preparing the compound of formula (1) comprises the following steps:

a) preparation of 4-fluoro-3-(1H-pyrrol-1-yl)nitrobenzene (10) by reacting 2-fluoro-5-nitroaniline with 2,5-dimethoxy- or 2,5-diethoxytetrahydrofuran in glacial acetic acid.This step of the process can be carried out in a polar environment althoug non-polar solvents can be also employed.The preferred solvents are : water,ethanol,dioxane,glacial acetic acid, N,N-dimethylformamide.As a catalyst one of the following substances can be used:acetic acid,hydrochloric acid,dry hydrogen chloride,p-toluene sulphonic acid....

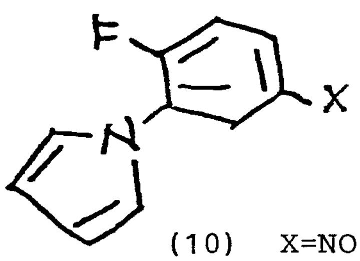

(10) $X=NO_2$

(11) $X=NH_2$

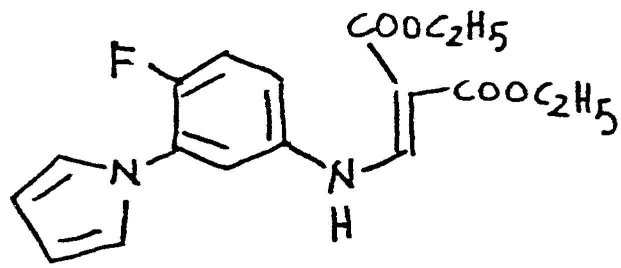

(12)

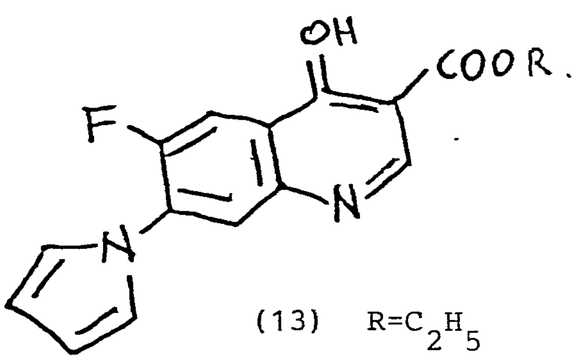

(13) $R=C_2H_5$

(14) R=H

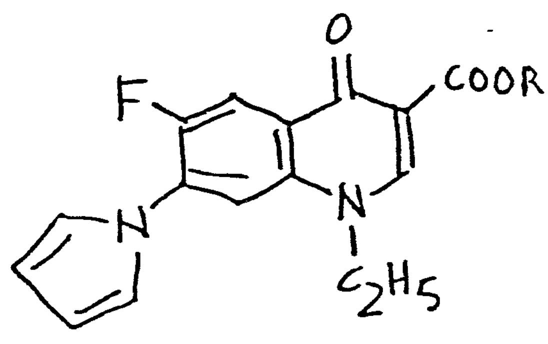

(15) $R=C_2H_5$

(1) R=H

b) reduction of 4-fluoro-3-(1*H*-pyrrol-1-yl)nitrobenzene (10) to 4-fluoro-3-(1*H*-pyrrol-1-yl)aniline (11) by hydrogenation in the presence of 5% palladium or 10% platinum on charcoal or 10% rhodium on alumina in an appropriate solvent (acetic acid,ethyl acetate, N,N-dimethylformamide).The hydrogenation can be carried out at 50-70°C under 4 atmospheres of pressure in a Parr apparatus.

c) treatment of 4-fluoro-3-(1*H*-pyrrol-1-yl) ani - line (11) with diethyl ethoxymethylenemalonate (EMME) at temperatures from 70°C to 180°C in absence of solvent or in the presence of protic or aprotic solvents,heating for 15 minutes or 4 hours.From this reaction diethyl 4-fluoro-3-(-1*H*-pyrrol-1-yl)anilinomethylenemalonate (12) formed.

d) cyclization of diethyl 4-fluoro-3-(1*H*-pyrrol-1-yl)anilinomethylenemalonate (12) to ethyl 6-fluoro-1,4-dihydro-4-oxo-7-(1*H*-pyrrol-1-yl)quinoline-3-carboxylic acid (13) by refluxing the former in diphenyl ether or similar boiling solvents (dowtherm,cellosolve, N,N-dimethylformamide...) for a time varying from 10 minutes to 3 hours.

e) treatment of ethyl 6-fluoro-1,4-dihydro--4-oxo-7-(1*H*-pyrrol-1-yl)quinoline-3-carboxylic acid (13) with ethyl iodide and sodium carbonate in N,N-dimethylformamide heating at 90°C for 4 hours to afford 1-ethyl-6-fluoro-1,4-dihydro-4-oxo-7-(1*H*-pyrrol-1-yl)quinoline-3-carboxylic acid ethyl ester (15).This ester was also obtained from ethylation of the acid (14),a product of alkaline hydrolysis of the corresponding ester (13).

f) alkaline hydrolysis of ethyl 1-ethyl-6-fluoro-1,4-dihydro-4-oxo-7-(1H-pyrrol-1-yl)quinoline-3-carboxylate (15) to afford the title 1-ethyl-6-fluoro-1,4-dihydro-4 - oxo-7-(1H-pyrrol-1-yl)quinoline-3-carboxylic acid (1) by refluxing the ester (15) for 1 hour in 10% aqueous sodium hydroxide.

Hydrogenation of (1) in the presence of 5% rhodium on alumina in glacial acetic acid gave the known acid (9).

The following procedures are intended to illustrate but not to limit,the scope of the present invention.

<u>4-fluoro-3-(1H-pyrrol-1-yl)nitrobenzene</u> (10)

A mixture of 15,6 g (0,1 mole) of 2-fluoro-5-nitroaniline, 13.2 g (0,1 mole) of 2,5-dimethoxytetrahydrofuran and 100 ml of glacial acetic acid was heated to 130°C for 30 minutes while stirring.After evaporation of the solvent under reduced pressure the residue was purified by passing it through a silica gel column (benzene as eluent).The collected eluates were evaporated to afford 18.2 g (88.3%) of 4-fluoro-3-(1H-pyrrol-1-yl)nitrobenzene, m.p.47-9° after crystallization from ligroin.

| | | | | | |
|---|---|---|---|---|---|
| | Found % | C 58.20; | H 3.40 | F 9.13 | N 13.41 |
| for | | | | | |
| $C_{10}H_7FN_2O_2$ calcd.: | | 58.25 | 3.43 | 9.21 | 13.59 |

Diethyl 4-fluoro-3-(1H-pyrrol-1-yl)anilinomethylenemalonate (12)

A solution of 4.12 g (0.02 mole) of (10) in 150 ml of 95° ethanol was hydrogenated in a Parr apparatus in the presence of 400 mg of 5% rhodium on alumina at the initial pressure of 4 atmosphere.When absorption of hydrogen stopped the mixture was filtered to remove the catalyst.The obtained solution was treated with 4.32 g (0.02 mole) of diethyl ethoxymethylenemalonate (EMME) and then submitted to evaporation at 70° under reduced pressure.The oily residue was heated to 130° for 30 more minutes and the solid which formed on cooling at room temperature was crystallized from ethanol to give 5.6 g (80.8% of the theoretical amount) of (12) m.p.86-8°.

| | | | | |
|---|---|---|---|---|
| Found %: | 52.30; | H 5.63 | F 5.37; | N 8.19 |
| for $C_{18}H_{19}FN_2O_4$ calcd.: | 62.41; | 5.54 | 5.49; | 8.09 |

I.R.spectrum: 1690 $cm^{-1}$ and 1640 $cm^{-1}$ ($COOC_2H_5$).

Ethyl 6-fluoro-4-hydroxy-7-(1H-pyrrol-1-yl)quinoline-3--carboxylic acid (13)

A suspension of 5.2 g (0,015 mole) of (12) in 30 ml of diphenyl ether was refluxed for 30 minutes.After cooling at room temperature n-hexane (200 ml) was added to the solution and the solid which precipitated was filtered,washed on the filter with diethyl ether and recrystallized from N,N-dimethylformamide to give 1.8 g (40.4%) of

(13),m.p. 312-5°.

| | | | | |
|---|---|---|---|---|
| Found%: | C 63.97; | H 4.30 | F 6.29 | N 9.50 |
| for $C_{16}H_{13}FN_2O_2$ calcd: | 63.99; | 4.37 | 6.33 | 9.33 |

6-Fluoro-4-hydroxy-7-(1H-pyrrol-1-yl)quinoline-3-carboxylic acid (14).

A suspension of 6.0 g (0.02 mole) of ester (13) in 30 ml of ethanol and 80 ml of 10% aqueous sodium hydroxide was heated at reflux for 1 hour.After cooling the solution was kept onto crushed ice (200 g) and made acidic with concentrated hydrochloric acid.The precipitate which formed was filtered and crystallized from N,N-dimethylformamide to yield 3.8 g (69.9%) of (14),m.p. 278-80°.

| | | | | |
|---|---|---|---|---|
| Found %: | 61.90; | H 3.41; | F 6.78; | N 10.22 |
| for $C_{14}H_9FN_2O_3$ calcd.: | 61.76 | 3.33 | 6.98 | 10.29 |

Ethyl 1-ethil-6-fluoro-1,4-dihydro-4-oxo-7-(1H-pyrrol--1-yl)quinoline-3-carboxylic acid (15).

METHOD A. A mixture of 6.0 g (20 mmoles) of (13), 2,8 g (20 mmoles) of finely powdered potassium carbonate and 6.2 g (40 mmoles) of ethyl iodide in 60 ml of N,N-dimethylformamide was heated at 90° for 4 hours while stirring. After cooling the mixture was poured onto crushed ice (200 g) and the precipitate which formed was filtered off and recrystallized from N,N-dimethylformamide to yield 4.0 g (60.9%) of (15),m.p. 223-6°.

Found %: C 65.99; H 5.16; F 5.89; N 8.80

for

| | | | | |
|---|---|---|---|---|
| $C_{18}H_{17}FN_2O_3$ calcd: | 65.84 | 5.22 | 5.79 | 8.53 |

I.R.spectrum:1720 $cm^{-1}$ ($COOC_2H_5$) and 1620 $cm^{-1}$ (CO).

METHOD B. A mixture containing 8.2 g (0.03 mole) of acid (14), 6.2 g (0.045 mole) of powdered potassium carbonate, 14.0 g (0.09 mole) of ethyl iodide and 120 ml of N,N-dimethylformamide was heated at 80-90° for 5 hours under stirring. After cooling the mixture was worked up as reported for method A. The yield was nearly 70%,calculated on recrystallized product.

<u>1-Ethyl-6-fluoro-1,4-dihydro-4-oxo-7-(1H-pyrrol-1-yl)quinoline-3-carboxylic acid</u> (1).

This compound was obtained from the corresponding ester (15) by alkaline hydrolysis following the procedure described for the acid (XXV) reported above. Yield: 73.3% after recrystallization from N,N-dimethylformamide; m.p 252-5°.

Found %: C 64.01; H 4.53; F 6.09; N 9.21

for

| | | | | |
|---|---|---|---|---|
| $C_{16}H_{13}FN_2O_3$ calcd.: | 63.99. | 4.36. | 6.33. | 9.33 |

I.R.spectrum:1720 $cm^{-1}$(COOH) and 1620 $cm^{-1}$(CO).

N.M.R.spectrum: $\delta$ 1.82(t;3H,$C\underline{H}_3-CH_2$), 4,98(q,2H,$CH_3-C\underline{H}_2$), 6.55 (m,2H,$\beta$-H pyrrole), 7.40 (m,2H,$\alpha$-H pyrrole), 8.27 (d,1H, $J_{H_8F}$=11 Hz,$J_{H_8H_5}$=0,$H_8$),8.58 (d,1H,$J_{H_5F}$=6 Hz,$J_{H_5H_8}$=0,

$H_5$) and 9.52 ppm (s,1H,$H_2$).

1-Ethyl-6-fluoro-1,4-dihydro-4-oxo-7-(pyrrolidin-1-yl) quinoline-3-carboxylic acid (9).

A mixture of 1.65 g (5.5 mmoles) of the acid (1) and 200 mg of 5% rhodium on alumina in 100 ml of glacial acetic acid was hydrogenated at 50° under 4 atmospheres of hydrogen for 6 hours.Removal of the catalyst by filtration and evaporation of the solvent *in vacuo* furnished the acid (9);yield:0.7 g (41.8%) after recrystallization from glacial acetic acid,m.p.330.1°

| | | | | |
|---|---|---|---|---|
| Found %: | C 63.12; | H 5.53; | F 6.09; | N 9.00 |
| for $C_{16}H_{17}FN_2O_3$ calcd.: | 63.14; | 5.63; | 6.24; | 9.21 |

I.R.spectrum: 1720 $cm^{-1}$ (COOH) and 1620 $cm^{-1}$ (CO)

N.M.R. spectrum: $\delta$ 1.73 (t,3H,$C\underline{H}_3$-$CH_2$), 2.20 (m,4H,$\beta$,$\beta'$--pyrrolidine methylene protons),3,87(m,4H,$\alpha$,$\alpha'$-pyrrolidine methylene protons),4.70 (q,2H,$CH_3$-$C\underline{H}_2$),6.90 (d,1H,$J_{H_8F}$=7 Hz,$H_8$),8.12 (d,1H,$J_{H_5F}$=14 Hz,$H_5$) and 9.15 ppm (s,1H,$H_2$).

## MICROBIOLOGICAL ASSAYS

### Materials and methods

Derivatives (1') and (9) were tested for the *in vitro* antibacterial activities against Gram-positive and Gram-negative bacteria.Nalidixic acid,pipemidic acid,-piromidic acid and enoxacin were used as positive controls for antibacterial activities.

Minimum inhibitory concentrations (M.I.C.s) were determined using the serial twolfold dilution method; lectures were performed after incubation at 37° for 18 hours.

For the experiments the substances were dissolved in NaOH 0.1 N(10 mg/ml);further dilutions in the test medium furnished the required concentrations, generally in the range from 0.05 μg/ml to 100 μg/ml.

The cultures were obtained on B.H.I. (B.B.L.) after 18 hours incubation at 37°.Tests were carried out using Mueller-Hinton agar (B.B.L.) and inocula were $10^4$ bacteria.

Media M.I.C. values $n\bar{X}$ ($c_{max}$ at least 200 μg/ml) were calculated by the equation

$$n\bar{X} = \frac{\sum_i (s_i \cdot c_i)}{s_t}$$

where $s_i$ is the number of sensitive strains at the given concentration $c_i$ and $s_t$ is the whole number of the sensitive strains.

Strains with M.I.C.> 200μg/ml are regarded as resistant (R) and are expressed in percentage (%) by the equation:

$$R\ (\%) = \frac{N_t - N_s}{N_t} \cdot 100$$

where $N_t$ is the whole number of tested strains and $N_s$ is

the number of sensitive strains.

The following species of bacteria freshly isolated from various clinical specimens were tested: 16 strains of Staphylococcus pyogenes,10 strains of Staphylococcus spp, 15 strains of Streptococcus ovalis,3 strains of Streptococcus spp,18 strains of Klebsiella pneumoniae,20 strains of Escherichia coli,11 strains of Salmonella spp (3 S.typhi,1 S.paratyphi A,S.paratyphi B, 4 S enteritidis, 1 S.wien and S.infantis,42 strains of Proteus spp (28 P.mirabilis, 8 P.vulgaris,3 P.rettgeri,2 P.morganii and 1 Providencia ) and 8 strains of Pseudomonas aeruginosa.

## Results

### Activity against Gram-positive bacteria.

The results of antimicrobial tests against Gram-positive bacteria showed that the activities of fluoroquinolonic acids,namely enoxacin,(1) and (9), were greatly superior to those of unfluorinated acids (nalidixic acid,pipemidic acid and piromidic acid).In fact,the absence of resistant strains (R%=0) and $n\bar{X}$ values are demonstrative of the higher activities of enoxacin (1) and (9).On the other hand comparison of the M.I.C. media values ($n\bar{X}$) relative to the activities of sensitive Gram-positive bacteria revealed the clear superiority of (1) and (9) with regard to enoxacin, the former being the best of all the compounds tested against Staphylococcus and Streptococcus.

### Activity against Enterobacteriaceae.

Against these Gram-negative bacteria the activities of (1) and (9) were found to show no remarkable

difference with enoxacin.Anyway the absence of resistant strains (R%=0) clearly shows that the acids (1) and (9) are more active than nalidixic acid,pipemidic acid and piromidic acid.

Acid (1) proved more active than enoxacin and (9) against Salmonella spp. and E.coli.

Activity against Proteus spp and Pseudomonas aeruginosa.

Compound (1) was found to be highly active against Proteus spp,more than all the compounds tested;also it was very active against Pseudomonas aeruginosa although not so active as enoxacin.These conclusions were derived from comparative examinations of R% and $n\bar{X}$ data.

**TABLE 1**

**Antibacterial activity of nalidixic, pipemidic and piromidic acids compared with the activity of norfloxacin and compounds (1) and (9) at pH 7.2 against 42 strains of PROTEUS spp (28 P.MIRABILIS, 8 P.VULGARIS, 3 P. RETTGERI, 2 P.MORGANII), 1 strain of PROVIDENCIA and 8 strains of PSEUDOMONAS AERUGINOSA.**

| Micro-organisms (n° of strains) | Nalidixic acid | | | Pipemidic acid | | | Piromidic acid | | | Norfloxacin acid | | | Acid (1) | | | Acid (9) | | |
|---|---|---|---|---|---|---|---|---|---|---|---|---|---|---|---|---|---|---|
| | R% | $n\bar{X}$ | range | R% | $n\bar{X}$ | range | R% | $n\bar{X}$ | range | R% | $n\bar{X}$ | range | R% | $n\bar{X}$ | range | R% | $n\bar{X}$ | range |
| Proteus spp (42) | 33 | 6.58 | 3.12- > 100 | 26 | 10.8 | 1.56- >100 | 76 | 60 | 25- >100 | 0 | 8.1 | 0.2- 100 | 0 | 6.46 | <0.1- 100 | 7 | 4.76 | 0.8- >100 |
| Pseudomonas aeruginosa (8) | 50 | 5.46 | 3.12- >100 | 0 | 12.3 | 1.56- 25 | 50 | 38.67 | 1.56- >100 | 0 | 1.42 | 0.4- 3.12 | 12.5 | 5.6 | <0.1- >100 | 12.5 | 9.87 | 0.2- >100 |

Reading was performed 18 h after incubation at 37°C.
R% = percentage of resistant strains.

$n\bar{X}$ = MIC mena values of sensitive strains.

TABLE II

Antibacterial activity of nalidixic, pipemidic and piromidic acids compared with the activity of norfloxacin and compounds (1) and (9) at pH 7.2 against 16 strains of STAPHYLOCOCCUS PYOGENES, 10 strains of STAPHYLOCOCCUS spp, 15 strains of STREPTOCOCCUS ovalis and 3 strains of STREPTOCOCCUS spp.

| Micro-organisms (n° of strains) | Nalidixic acid | | | Pipemidic acid | | | Piromidic acid | | | Norfloxacin | | | Acid (1) | | | Acid (9) | | |
|---|---|---|---|---|---|---|---|---|---|---|---|---|---|---|---|---|---|---|
| | R% | $n\bar{X}$ | range | R% | $n\bar{X}$ | range | R% | $n\bar{X}$ | range | R% | $n\bar{X}$ | range | R% | $n\bar{X}$ | range | R% | $n\bar{X}$ | range |
| Staphylococcus pyogenes (16) | 94 | 100 | 100- >100 | 44 | 36.4 | 3.12- >100 | 69 | 11.2 | 3.12- >100 | | 8.64 | 0.4- 25 | | 1.96 | 0.1- 12.5 | | 5.95 | 0.4- 25 |
| Staphylococcus spp (10) | 50 | 25.6 | 3.12- >100 | 30 | 39.06 | 1.56- >100 | 60 | 39.06 | 1.56- >100 | | 4.79 | 0.2- 25 | | 1.26 | 0.1- 3.12 | | 3.98 | 0.8- 6.25 |
| Streptococcus ovali (15) | 93 | 3.12 | 3.12- >100 | 66 | 65.6 | 3.12 >100 | 87 | 100 | 100- >100 | | 17.2 | 0.8- 100 | | 8.4 | 0.8- 100 | | 6.2 | 3.12- 25 |
| Streptococcus spp (3) | 100 | | >100 | | 75 | 25- 100 | | 10.4 | 6.25- 12.5 | | 3.6 | 1.56- 6.25 | | 0.2 | 0.2 | | 2.2 | 0.4- 3.12 |

Reading was performed 18 h after incubation at 37°C
R% = percentage of resistant strains; $n\bar{X}$ = MIC mean values of sensitive strains.

TABELLA III

Antibacterial activity of Nalidixic, pipemidic and piromidic acids compared with the activity of norfloxacin, acid (1) and acid (9) at pH 7.2 against 18 strains of KLEBSIELLA PNEMONIAE, 20 strains of E.COLI,11 strains of SALMONELLA spp (4SALMONELLA ENTERITIDIS, 3 SALMONELLA TYPHI, 1 SALMONELLA WIEN, 1 SALMONELLA PARATYPHI A, 1 SALMONELLA PARATYPHI B, 1 SALMONELLA INFANTIS).

| Micro-organisms (n° of strains) | Nalidixic acid R% n$\bar{X}$ range | Pipemidic acid R% n$\bar{X}$ range | Piromidic acid R% n$\bar{X}$ range | Norfloxacin R% n$\bar{X}$ range | Acid (1) R% n$\bar{X}$ range | Acid (9) R% n$\bar{X}$ range |
|---|---|---|---|---|---|---|
| Klebisella pneumoniae | 22 12.9 3.12- >100 | 22 3.79 1.56- >100 | 61 714 50- >100 | 1.93 0.2- 6.25 | 3.4 0.8- 25 | 12.67 3.12- 25 |
| E.Coli | 25 12.39 1.56 >100 | 30 4.12 1.56- >100 | 45 44.3 6.25- >100 | 4.47 0.2- 25 | 2.0 0.2- 12.5 | 10 3.83 0.8- >100 |
| Salmonella spp (11) | 18 5.2 3.12- >100 | 9 17.6 1.56- >100 | 36 60.93 1.56- >100 | 2.66 0.2- 12.5 | 1.3 <0.1- 3.12 | 2.78 0.2- 6.25 |

Reading was performed 18 h after incubation at 37°C.
R% = percentage of resistant strains n$\bar{X}$ = MIC mean values of sensitive strains.

CLAIMS:

1. A compound having the formula (1)

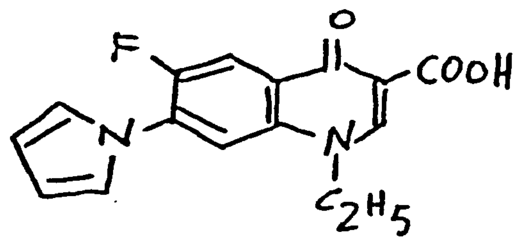

(1)

2. A process for preparing the compound of claim 1 which comprises:

a) reaction of 4-fluoro-3-aminonitrobenzene with 2,5 dimethoxytetrahydrofuran in glacial acetic acid to afford 4-fluoro-3-(1H-pyrrol-1-yl)nitrobenzene (2)

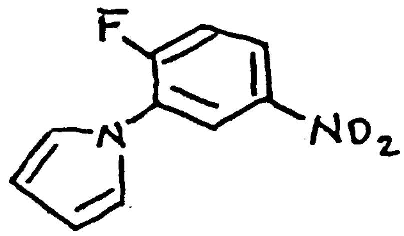

(2)

b) reduction of 4-fluoro-3-(1H-pyrrol-1-yl)nitrobenzene to 4-fluoro-3-(1H-pyrrol-1-yl)aniline (3)

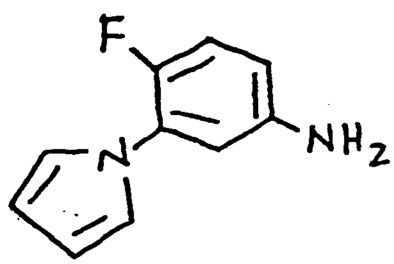

(3)

by hydrogenation in the presence of a catalyst (5% palladium or 10% platinum on charcoal:10% rhodium on alumina)in a suitable solvent (acetic acid,ethyl acetate);

c) treatment of 4-fluoro-3-(1H-pyrrol-1-yl)aniline with diethyl ethoxymethylenemalonate (EMME) to obtain diethyl 4-fluoro-3-(1H-pyrrol-1-yl)anilinomethylenemalonate (4)

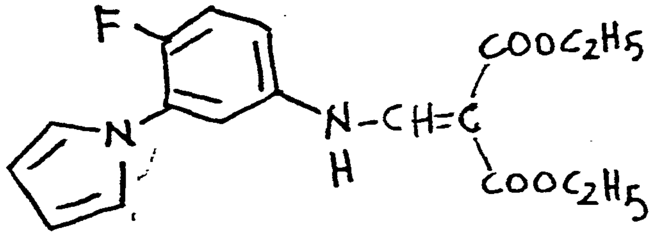

(4)

d) cyclization of diethyl 4-fluoro-3-(1H-pyrrol-1-yl)anilinomethylenemalonate by refluxing in diphenyl ether to obtain ethyl 6-fluoro-1,4-dihydro-4-oxo-7-(1H-pyrrol-1-yl)quinoline-3-carboxylic acid (5)

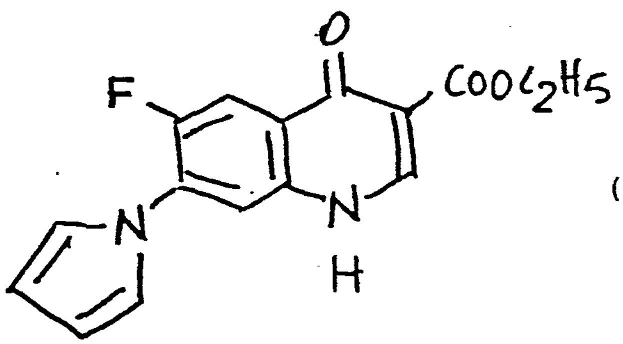

(5)

e) treatment of ethyl 6-fluoro-1,4-dihydro-4-oxo-7-(1H-pyrrol-1-yl)quinoline-3-carboxylic acid with ethyl iodide and sodium carbonate in N,N-dimethylformamide to give 1-ethyl-6-fluoro-1,4-dihydro-4-oxo-7-(1H-pyrrol-1-yl)-quinoline-3-carboxylic acid ethyl ester (6)

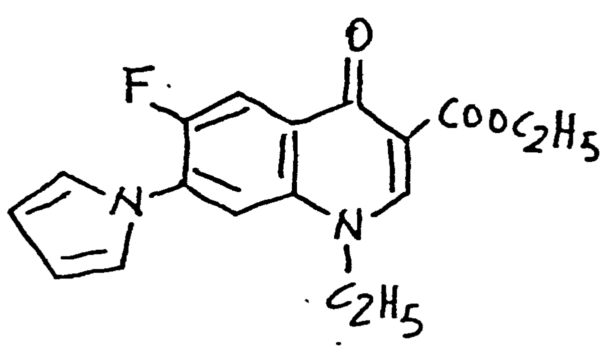

(6)

f) alkaline hydrolysis of ethyl 1-ethyl-6-fluoro--1,4-dihydro-4-oxo-7-(1H-pyrrol-1-yl)quinoline-3-carboxylate to obtain the 1-ethyl-6-fluoro-1,4-dihydro-4-oxo--7-(1H-pyrrol-1-yl)quinoline-3-carboxylic acid (1) of the claim 1.

3) A process for preparing the compound of the claim 1 which comprises :

a) ethylation of compound (3) of claim 2 to obtain N-ethyl 4-fluoro-3-(1H-pyrrol-1-yl)aniline (7)

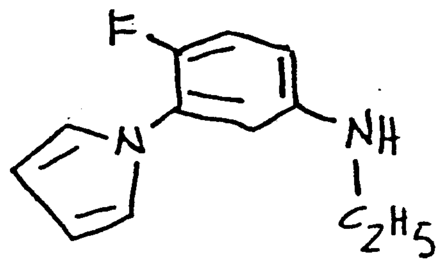

(7)

b) treatment of compound (7) with EMME to afford N-ethyl diethyl 4-fluoro-3-(1H-pyrrol-1-yl)anilinomethylenemalonate (8)

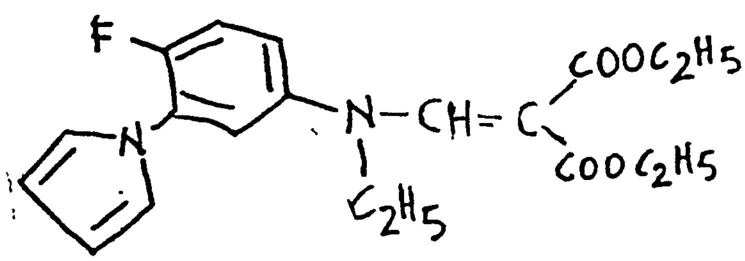

(8)

c) cyclization of compound (8) to the ethyl ester (6) of claim 2;

d) hydrolysis of the ester (6) to the acid (1) of claim 1.

4. A process for preparing the compound (1) of the claim 1 based on:

a) hydrolysis of the ester (5) of the claim 2 to obtain 6-fluoro-1,4-dihydro-4-oxo-7-(1H-pyrrol-1-yl) quinoline-3-carboxylic acid (9)

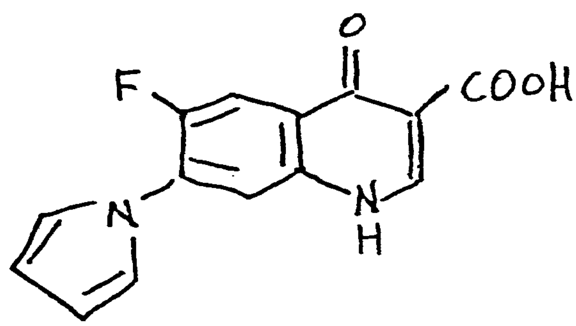

(9)

b) ethylation of the acid (9) to afford the ester (6) of the claim 2;

c) hydrolysis of the ester (6) to furnish the acid (1) of the claim 1.

5. An orally or parenterally administrable pharmaceutical composition comprising a therapeutically effective amount of the acid (1) of claim 1 and a pharmacologically acceptable excipient therefor.

6. The composition of claim 5 for the therapeutical treatment of a patient in need of an antimicrobial pharmaceutical composition to cure topical or systemic infections from pathogen micro-organisms.